# EUROPEAN PATENT APPLICATION

(11) **EP 4 111 958 A2**
(43) Date of publication of application: **04.01.2023**
(21) Application number: 22181882.6
(22) Date of filing: 29.06.2022
(51) Int. Cl.: A61B 5/08, G01N 33/497, A61B 5/083, A61B 5/20

(54) **ELECTROCHEMICAL SENSING FOR BREATH ANALYSIS**

(30) Priority: 30.06.2021 US 202163217275 P
(71) Applicant: Heteron Biotechnologies, LLC, Setauket, New York 11733 (US); Rigas, Anastasia, Setauket, New York 11733 (US)
(72) Inventor: RIGAS, Anastasia, Setauket, 11733 (US); ARTAN, Nabi Sertac, Brooklyn, 11223 (US); AMOAKO, Edward, Corona, 11368 (US); CHIANG, Hao-Chun, Syosset, 11791 (US)
(74) Representative: Herrero & Asociados, S.L.

(57) **Abstract**

The present invention provides an improved breath analyzers and breath test methods to determine the presence of diseases such as gastrointestinal, liver, kidney, and metabolic diseases.

## Description

### FIELD

**0001** The present application relates generally to breath analyzer and breath test method for detecting gases in the breath of subjects with diseases such as liver diseases, kidney diseases, metabolic diseases, carbohydrate malabsorption, small bowel intestinal overgrowth, H. Pylori infection and others.

### PRIORITY

**0002** This application claims priority to provisional application No. 63/217,275, which is herein incorporated by reference.

### BACKGROUND

**0003** Non-alcoholic steatohepatitis (NASH) is part of the spectrum of non-alcoholic fatty liver disease (NAFLD) which has become the most common liver disease in the world due to worldwide increasing rates of obesity and metabolic syndrome. NASH develops when lipotoxic lipids, accumulated due to fatty infiltration of the liver, cause significant hepatocellular injury.

**0004** Lipotoxic lipids (diacylglycerols, ceramides and others) mediate an array of intracellular processes (endoplasmic reticulum stress, mitochondrial dysfunction, inflammation and apoptosis) to produce the histologic phenotype of NASH. These intracellular processes are the stimuli for fibrogenesis and possibly hepatocellular carcinoma (HCC).

**0005** An estimated 2%-5% of the US population or 20% of the population with obesity-induced NAFLD will advance to NASH and cirrhosis without clearly established predictability criteria, and NASH may soon become the leading indication for liver transplantation. The cumulative annual risk for developing HCC in patients with NASH-fibrosis, if untreated, ranges from 2.4% to 12.8% and the projected annual economic impact of NAFLD-NASH is estimated at $103 billion in the US. There is a dire need to identify and treat NASH before the development of cirrhosis.

**0006** Liver biopsy, an invasive procedure, is the gold standard in diagnosis and staging of NASH fibrosis along with elevated serum alanine transferase (ALT) ≥50 IU/L and imaging (ultrasonography-MRI) studies. Biomarkers are expected to improve the ability to stratify disease severity in NAFLD and to identify additional pathways to target for treatment before it advances to NASH fibrosis and cirrhosis. Rigorous review of the literature reveals that several non- invasive biomarkers and panels of biomarkers with blood tests that reflect underlying disease pathways in NASH are being developed.

**0007** Biomarkers such as Caspase-generated CK-18 fragment (CK-18), fibroblast growth factor 21(FGF21), insulin-like growth factor 2 (IGF-2) as well as epidermal growth factor receptor (EGFR) have moderate diagnostic and prognostic accuracy.

**0008** Biomarker panels such as AST:ALT ratio, NAFLD fibrosis score, BARD score (BMI, AST:ALT ratio, Diabetes mellitus) are less accurate than specific fibrosis markers (FibroTest, FibroMeter). These specific fibrosis markers accurately predict the fibrosis stage, only, after fibrosis has long been developed. Hyaluronic acid (HA), a major component of extracellular matrix, is detected during advanced stages of fibrosis.

**0009** Imaging biomarkers such as FibroScan and point shear wave elastography (psWE) have moderate to high accuracy of diagnosing advanced fibrosis or cirrhosis but not early stages of fibrosis. Magnetic Resonance Elastography (MRE) has higher success rate and accuracy than ultrasound-based technologies but is limited by cost and availability.

**0010** Genetic and genomic markers for assessment of disease susceptibility and disease severity are being developed but with limitations in accuracy and reproducibility with regards to prediction of NAFLD and NASH and its severity.

**0011** Metabolomic studies are underway and studies of the microbiome profile produced an algorithm that could predict advanced fibrosis suggesting that a test based on it would be a useful marker. However, such marker would not predict early fibrogenesis.

**0012** None of the existing biomarkers is a breath test, the simplest method of testing for a potential biomarker for NASH. Recent, compelling evidence in animal studies and in humans shows that hepatocellular injury causes structural changes (gene expression, activity) in the urea cycle enzymes ornithine transcarbamylase (OTC) and carbamoyl phosphate synthetase (PS), thus impairing ammonia's conversion to urea resulting in increased ammonia (hyperammonemia).

**0013** So far, despite promising clinical studies, an accurate, reproducible non-invasive method to predict early NASH has not been identified. There is a need for accurate, non-invasive, reproducible, and specific biomarker with high predictive value of NASH fibrosis at the earliest possible stage of fibrogenesis.

**0014** Studies have shown a direct link between increased ammonia concentration and the development of NASH fibrosis which can lead to cirrhosis, end stage liver disease and HCC. Ammonia at concentrations of 50-300 µM/L activates the usually quiescent hepatic stellate cells (HSC) which then become highly proliferative and synthesize a fibrotic matrix rich in type I collagen becoming key for the development of fibrosis, portal hypertension and HCC. Hyperammonemia due to hepatocellular injury is among the earliest stimuli for the development of fibrosis in NASH.

**0015** The toxicity of hyperammonemia, especially on the central nervous system, makes monitoring of ammonia a priority in patients predisposed to developing hyperammonemia. However, measurement of blood ammonia is considered non-reliable, as per AASLD, due to inaccuracies associated with blood drawing methods (arterial blood ammonia is preferable but the procedure is painful and requires professionals to draw, while venus blood often gives inaccurate ammonia results) and errors with the transport of the specimen to the lab.

**0016** Exhaled breath consists of 78% N₂, 13% O₂, 4%-5% CO₂ and traces of gases such as ammonia, acetone and other volatile organic compounds (VOCs). Measurement of breath ammonia, a simpler and more accurate procedure than blood ammonia, can replace measurement of blood ammonia especially since ammonia is in equilibrium in the lungs and studies have shown that blood ammonia correlates with breath ammonia.

**0017** Several instruments are used in laboratory setting for detection of ammonia in breath. They include Gas Chromatography-Mass Spectrometry (GC-MS), Atmospheric Pressure Chemical Ionization-Mass Spectroscopy (APCI-MS), Selected Ion Flow Tube Mass-Spectrometry (SIFT-MS), Laser Spectroscopy and Laser Photoacoustic Spectroscopy (LPAS). Such instruments, however, are large, complex, very expensive and require concentrated breath for accurate measurement.

**0018** With the exception of SIFT-MS, these technologies are not reliable since breath is often collected in plastic bags or balloons, transported and injected into the instruments. This leads to potentially inaccurate results due to contamination or damage of the breath sample during transport. Even though SIFT-MS is highly sensitive to ammonia (10ppb), it is an expensive and complex instrument, must be operated by professionals, and is not available at POC or for home testing.

**0019** Optical, chemical and electrochemical sensor systems for detection of ammonia at very low concentrations (50ppb and below) have also been developed but none has reached commercialization; Quartz crystal microbalance sensors, metal oxide (MoO3) sensors and electronic nose (E-nose) systems have not been successful as they are unstable, require extremely high temperatures (MoO3) to operate and are mostly qualitative (E-nose).

**0020** A non-invasive, simple and accurate breath test method to measure ammonia will prevent the damaging effect of hyperammonemia in NASH fibrosis and will monitor the central nervous system toxicity in chronic liver disease, chronic kidney disease, urea cycle defect and other metabolic diseases. **0021** Small Intestinal Bacterial Overgrowth (SIBO) is defined as the presence of bacteria in the small intestine where bacteria should not be present. This intestinal abnormality presents with abdominal pain, diarrhea, weight loss and malabsorption of nutrients such as carbohydrates and others. The presence of bacteria in the small intestine contribute to the development of inflammation which further exacerbates the symptoms.

**0022** Diagnosis of SIBO is obtained through bacterial cultures of small intestinal aspirant and through breath test for hydrogen and/or methane. Identification of the type of bacteria present in the small intestine can lead to successful treatment. However, aspiration of the luminal content (fluid) of the small intestine requires an invasive procedure, upper GI endoscopy, which carries risks (sedation for the procedure, perforation of the gastrointestinal tract, bleeding) and which may not always achieve the objective due to low yield of aspiration of intestinal fluid.

**0023** Breath test for diagnosis of SIBO has been standard practice by health care providers. The breath test is used to measure hydrogen (H₂) and/or methane (CH₄) in breath of individuals presumed to have SIBO. The existing standard breath test is performed using equipment such as gas chromatography and mass spectroscopy. The standard test requires the tester to fast for several hours and at the end of the fast to ingest a known quantity of carbohydrate solution. Prior to ingestion, the subject exhales into a bag (baseline breath sample) and after the ingestion the subject exhales into another bag several times in 20-30-minute intervals for up to 2 hours post ingestion. If the post ingestion sample contains higher amount of hydrogen and/or methane than the baseline breath sample in 30-60 minutes post ingestion, then the subject has evidence of malabsorption of carbohydrate which can be consistent with SIBO. The earlier the increase of hydrogen and/or methane post ingestion, the higher the probability of SIBO.

**0024** Methane can be detected via gas chromatography using a variety of detectors. These include flame ionization detectors, thermal conductivity detectors, pulsed helium discharge ionization detectors and mass spectrometry. Breath methane has also been measured in humid atmospheres using selective ion flow transfer mass spectrometry (SIFT- MS). A number of simple sensors such as those based on metal oxides, catalytic based sensors such as pellistors or other catalytic sensors, semistors and piezoelectric sensors and SAW devices have been deployed in the detection of methane.

**0025** New materials such as carbon nanotubes have been utilized to enable room temperature detection of methane. In addition to these solid-state sensors, a number of electrochemical sensors have been used to detect methane; these include amperometric sensors and methane biosensors which utilize methanotrophic bacteria and also methane fuel cells. A recent review of near infrared methane detection methods based on tunable diode lasers with comparison of detection limits has been described. Many methane detectors are designed for environmental monitoring or as leak detectors where low limits of detection are not required.

**0026** Commercial instruments which are capable of simultaneous measurements of methane and hydrogen in breath are available, specifically designed for monitoring carbohydrate malabsorption syndromes or SIBO. The Quintron BreathTrackerTM SC separates the gas components (H₂ and CH₄) by the basic principle of gas chromatography, using room air as the carrier gas, which is pumped through the system by an internal circulating pump. Hydrogen and methane are separated from all other reducing gases and from each other, and are carried past a solid-state sensor. Lactotest 202 instrument uses an electrochemical hydrogen sensor and an infrared sensor for methane and carbon dioxide detection. There is also the GastroCH4ECK^{™} instrument which simultaneously measures hydrogen, methane and oxygen in breath by using an infrared sensor for methane and an electrochemical sensor for hydrogen.

**0027** Vanadium (IV) oxide (VO₂) gas sensor for methane. Researchers have applied the monoclinic and tetragonal rutile phases of VO₂ for sensing gases such as CH₄, H₂, CO₂ and CO using a first principles method. Based on their results and simulations, VO₂ shows a highly selective sensing performance towards CH₄ compared to H₂, CO and CO₂ gases.

**0028** Methane tends to form stable H-O bonds with the monoclinic and tetragonal rutile phases of VO₂, with lengths of 0.95 A and 1.05 A, which are much shorter than the adsorption distances found for other gases, revealing a chemical adsorption characteristic for methane. The maximum current in the current-voltage relationship obtained for the VO₂ (R)-CH₄ system reach 8.0 µA (1.5V). The diffusion of methane gas in VO₂ with monoclinic and tetragonal rutile phases is more difficult compared to other gases due to chemical adsorption of methane.

**0029** There is a range of methods for detection of methane, from conventional gas chromatography methods to spectroscopic techniques developed for atmospheric monitoring. Some of these techniques have been or could be applied to monitoring methane levels in breath. For breath methane detection, methods of detection which can detect methane from 1-100 ppm taking into account the atmospheric methane which is about 1.7 ppm.

**0030** Methane can be detected via gas chromatography using a variety of detectors. These include flame ionization detectors, thermal conductivity detectors, pulsed helium discharge ionization detectors and mass spectrometry. Breath methane has also been measured in humid atmospheres using selective ion flow transfer mass spectrometry (SIFT- MS). A number of simple sensors such as those based on metal oxides, catalytic based sensors such as pellistors or other catalytic sensors, semistors and piezoelectric sensors and SAW devices have been deployed in the detection of methane.

**0031** New materials such as carbon nanotubes have been utilized to enable room temperature detection of methane. In addition to these solid-state sensors, a number of electrochemical sensors have been used to detect methane; these include amperometric sensors and methane biosensors which utilize methanotrophic bacteria and also methane fuel cells. A recent review of near infrared methane detection methods based on tunable diode lasers with comparison of detection limits has been described. Many methane detectors are designed for environmental monitoring or as leak detectors where low limits of detection are not required. There are relatively few reports of methane detectors used for breath analysis in humans or animals. A summary is provided in Table 1.

| **Table 1.** Comparison of different methane sensors. | | | | |
|---|---|---|---|---|
| **Methane Sensor Types** | **Working Mechanisms** | **Advantages** | **Disadvantages** | **Related Research** |
| Optical sensors | Detect changes in light waves that result from an interaction of the analyte with the receptor part. | Non-destructive method; Immune to electromagnetic interference; Operate without oxygen | High cost; High power consumption; Lack of significance and distinctiveness of methane optical absorption region. | [28,30-35] |
| Calorimetric sensors | Measure the heat produced from a reaction and correlate the value to the reactant concentration. | Low cost; Simplistic design; Portable; Easy to manufacture; Good selectivity for methane; Can operate in harsh environmental conditions. | Low detection accuracy; Susceptible to cracking, catalyst poisoning and oversaturation; High power consumption; Short lifespan, Require high temperature. | [40,48-52] |
| Pyroelectric sensors | Convert thermal energy into electrical energy based on the phenomenon of pyroelectricity. | Non-destructive; Operate without oxygen, Good sensitivity and responsivity; Wide measuring range; Operate at room temperature. | High cost; High power consumption; [57,58,64-67] Immobile; Difficult to manufacture. | |
| Semiconducting metal oxide sensors | Absorption of gas on the surface of a metal oxide changes its conductivity, which is then quantified to obtain the gas concentration. | Low cost; Lightweight and robust; Long lifespan; Resistant to poisoning. | Poor selectivity; Small and high operational temperature range; Slow recovery rate; Significant additive dependency; Affected by temperature; Susceptible to degradation; Sensitive to changes in humidity | [77-83] |
| Electrochemical sensors | Measure the target gas concentration by oxidizing or reducing the gas at an electrode and measuring the resulting current. | AE-based: Low cost. IL-based: Non-hazardous materials; High boiling points and low volatility; Good selectivity for methane; Can detect small leak SE-based: No leakage; Safe; Robust; Good selectivity for methane; Can detect small leaks. | AE-based: Susceptible to leakage and evaporation, Hazardous materials; Slow response time. IL-based: Susceptible to leakage; Slow response time. SE-based: Require high temperature; Unable to detect low gas concentrations; Susceptible to degradation or loss of electrolyte. | [97,100,106-111] |

### BRIEF DESCRITION OF DRAWINGS

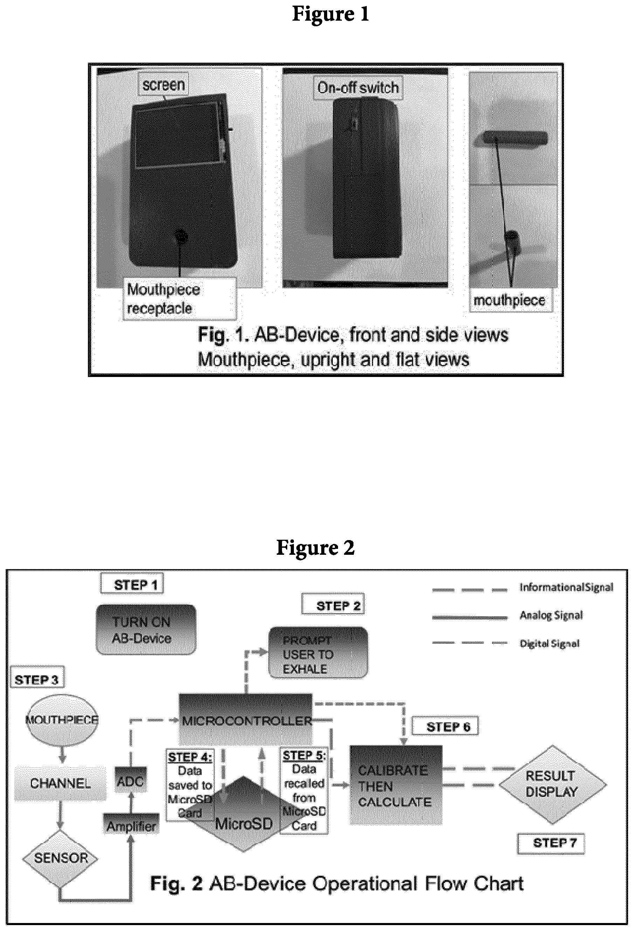

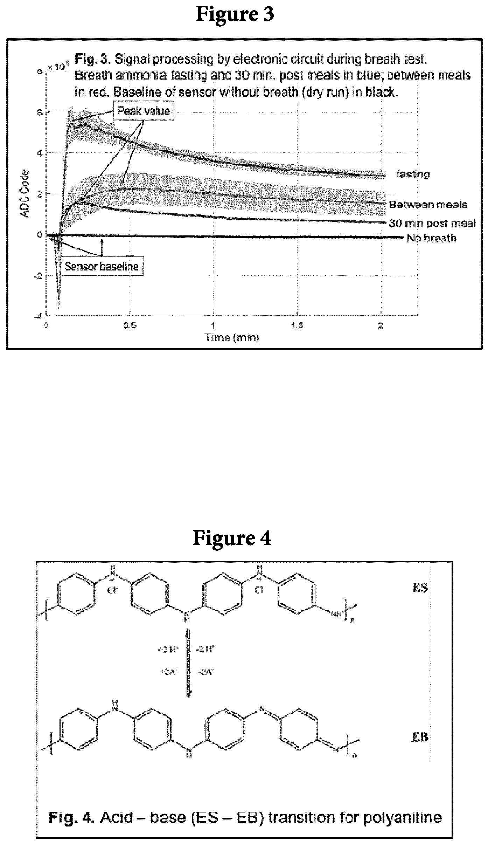

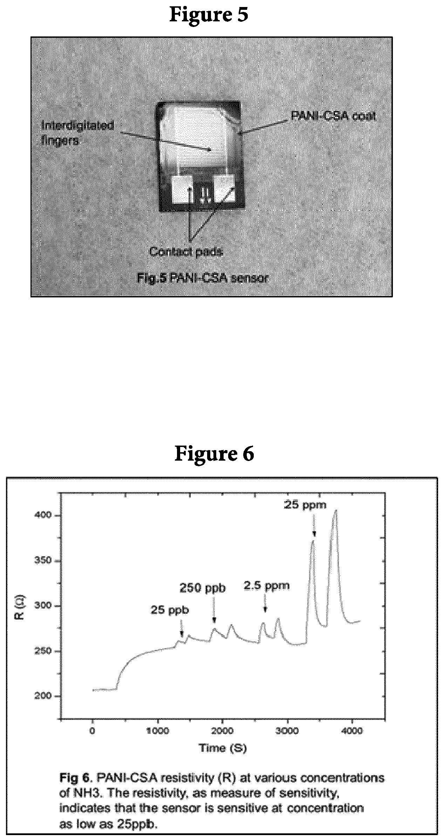

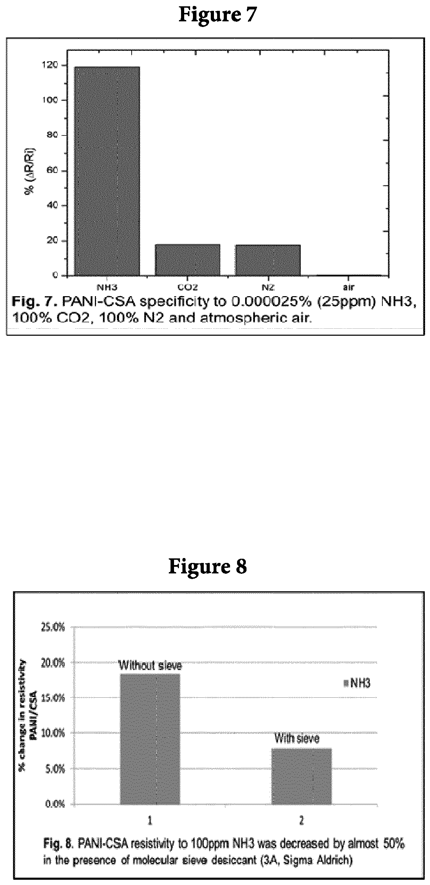

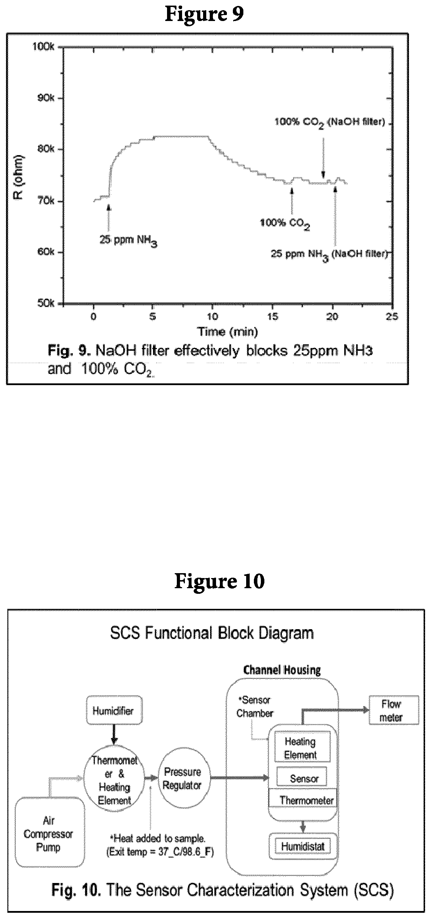

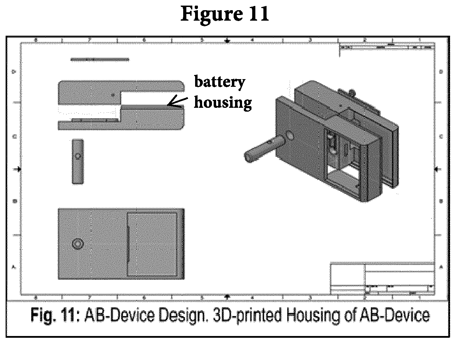

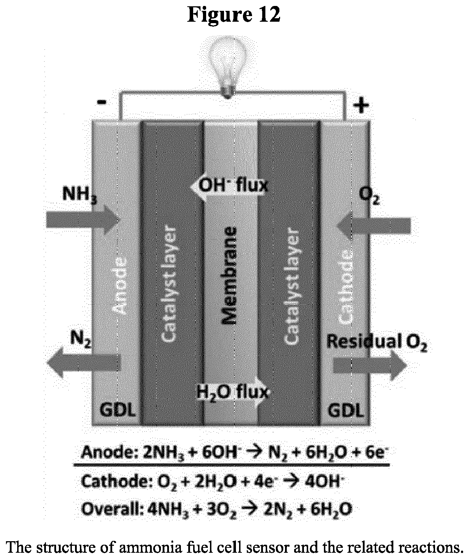

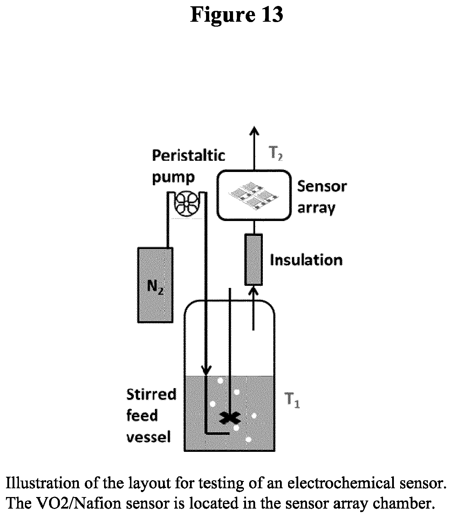

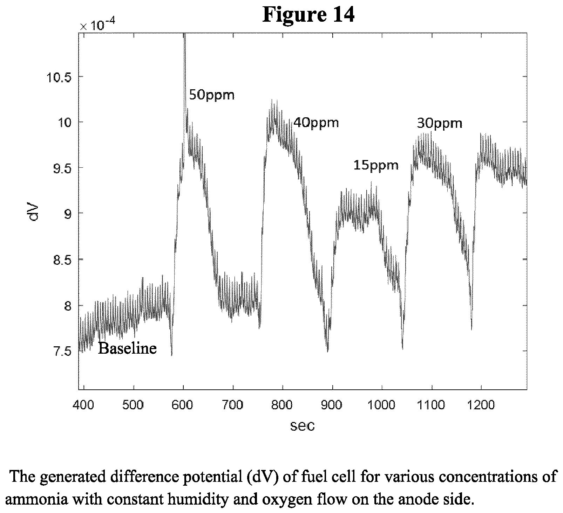

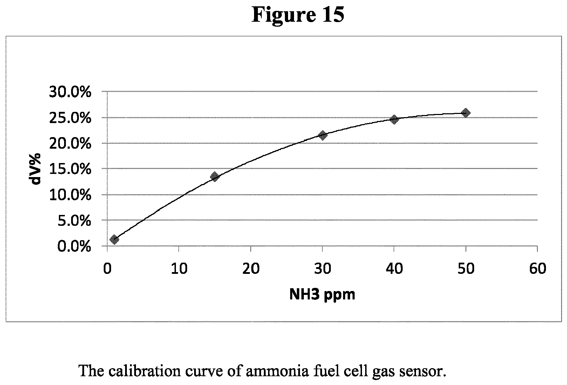

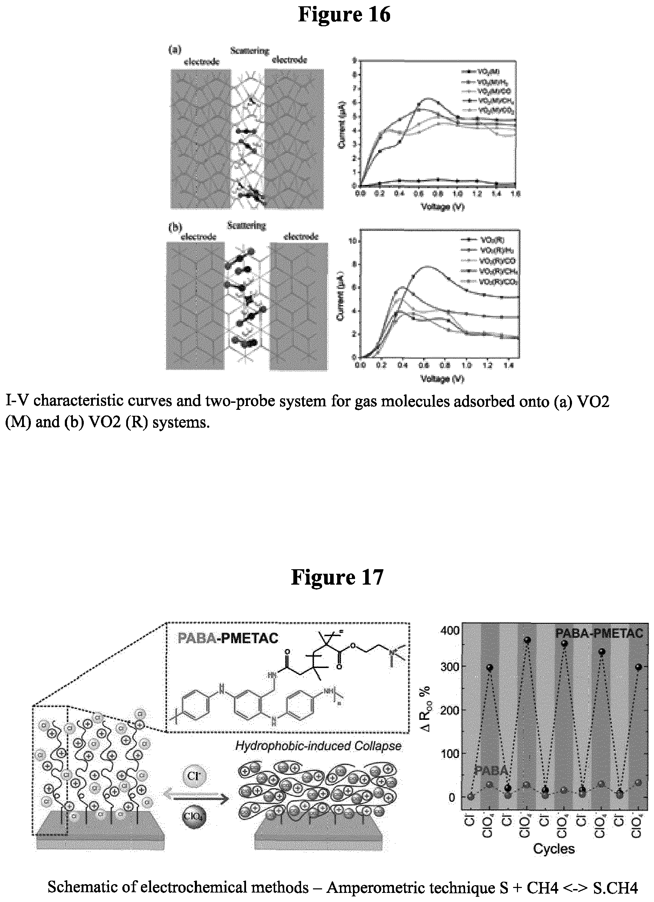

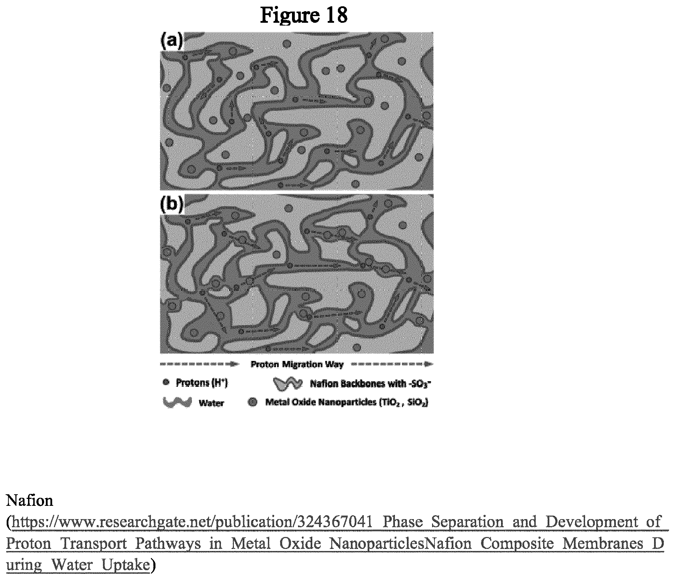

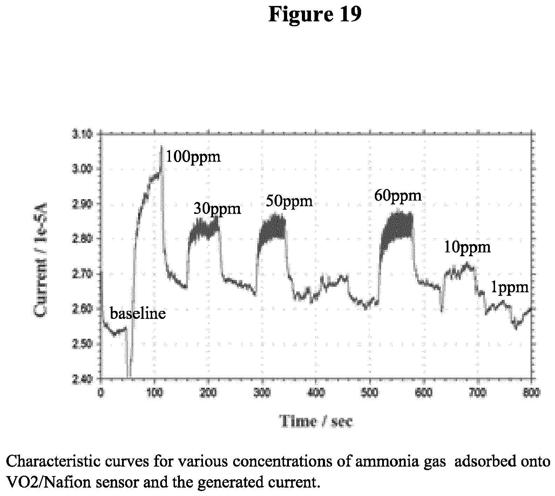

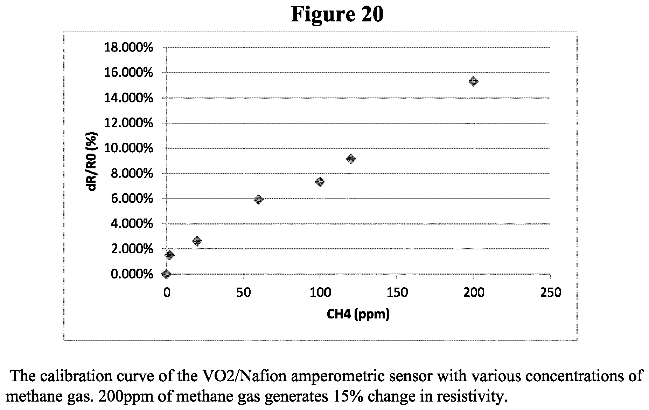

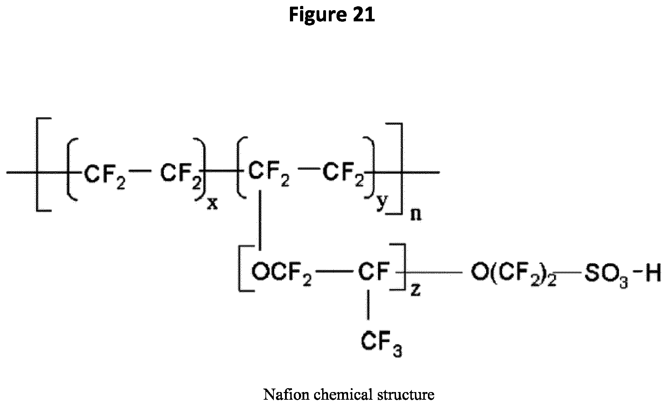

0032 The following drawings are illustrative of particular examples of the present invention and therefore do not limit the scope of the invention. The drawings are not to scale (unless so stated) and are intended for use in conjunction with the explanations in the following detailed description. Examples of the present invention will hereinafter be described in conjunction with the appended drawings, wherein like numerals denote like elements.
**0033** Figure 1 illustrates a breath analyzer device, which is an example of ammonia breathalyzer device (AB-Device), with front and side views and the removable mouthpiece;
**0034** Figure 2 illustrates the operational flow of the breath analyzer device, which is an example of the operational flow when the sensor is an ammonia sensor;
**0035** Figure 3 illustrates examples of analysis of the ammonia sensor signal when the subjects exhale breath samples into the mouthpiece using the ammonia breath analyzer. The graph labeled "fasting" illustrates the analysis of the signal from the ammonia sensor when the subject exhales into the breath analyzer after overnight (9 hours) fasting. The graph labeled "between meals" illustrates the analysis of signal from the ammonia sensor when the subject exhales into the breath analyzer at half time between two meals. The graph labeled "30 min post-meal" demonstrates the analysis of signal from the ammonia sensor when the subject exhales into the breath analyzer 30 minutes after ingestion of the first post-fasting meal. The straight line illustrates the baseline of the ammonia sensor without the subject exhaling into the device (no breath). The straight line demonstrates the stability of the ammonia sensor's conductance.
**0036** Figure 4 illustrates an acid - base (emeraldine salt (ES) - emeraldine base (EB)) transition for polyaniline;
0037 Figure 5 illustrates the ammonia PANI-CSA sensor;
**0038** Figure 6 illustrates the ammonia sensor (PANI-CSA) resistivity after exposure of various concentrations of ammonia on the sensor;
**0039** Figure 7 illustrates the PANI-CSA sensor specificity to CO₂, N₂ and air;
**0040** Figure 8 illustrates the effect of the molecular sieve desiccant (3A, Sigma Aldrich) on the resistivity of the PANI-CSA sensor;
**0041** Figure 9 illustrates the effect of NaOH filter on ammonia (NH₃) and the resistivity of the PANI-CSA sensor;
**0042** Figure 10 illustrates the sensor characterization system (SCS);
**0043** Figure 11 illustrates the 3-D printed housing of the AB-Device;
**0044** Figure 12 illustrates the structure of the fuel cell ammonia sensor and the related chemical reactions;
**0045** Figure 13 illustrates the layout of a system for testing an electrochemical sensor;
**0046** Figure 14 illustrates the generated electrical potential difference of fuel cell sensor for various concentrations of ammonia with constant humidity and oxygen flow on the anode side;
**0047** Figure 15 illustrates the calibration curve of ammonia detected using a fuel cell sensor;
**0048** Figure 16 illustrates characteristic curves (I-V) and two-probe system for gas molecules adsorbed onto (a) VO₂ (M) and (b) VO₂ (R) systems;
**0049** Figure 17 illustrates electrochemical methods for methane (CH₄) gas sensing;
**0050** Figure 18 illustrates Nafion and its properties when enhanced with metal oxides like TiO₂ and SiO₂;
**0051** Figure 19 illustrates characteristic changes in current (A) when methane gas molecules are adsorbed onto VO₂/Nafion amperometric sensor; and
**0052** Figure 20 illustrates the calibration curve of the amperometric methane sensor when the sensor is exposed to various concentrations of methane gas.
**0053** Figure 21 illustrates the chemical structure of Nafion.

### SUMMARY

**0054** The present invention provides breath analyzer and breath test methods to determine the presence of gases in breath as they relate to liver disease, kidney disease, metabolic disease, intestinal carbohydrate malabsorption, H. Pylori infection, small intestinal bacterial overgrowth (SIBO) and others.

**0055** Some embodiments provide a hand-held, portable, breath analyzer comprised of a removable mouthpiece, and a body which contains a channel, a sensor, an amplifier, an analog to digital converter (ADC), a microcontroller, a microSD card, a liquid-crystal display (LCD) and a power source. In some cases, the sensor comprises an ammonia selective material and a conductive material, wherein the ammonia selective material contacts the conductive material, wherein the ammonia selective material is doped polyaniline and has a resistivity that increases in response to increased concentration of ammonia. The microcontroller detects resistivity and uses the resistivity to calculate a concentration of ammonia in the breath sample which is displayed on the screen. The conductive material can include a plurality of electrodes, e.g. interdigitated finger electrodes.

**0056** Some embodiments provide a hand-held, portable, breath analyzer including a removable mouthpiece, and a body which contains a sensor, a channel, an amplifier, an analog to digital converter (ADC), a microcontroller, a microSD card, a liquid-crystal display (LCD) and a power source. The power source is a battery, wherein the battery is a 9V battery or a lithium-polymer rechargeable battery; The sensor comprises ammonia selective material that has a resistivity that increases in response to increased concentration of ammonia (e.g., polyaniline (PANI) doped with a dopant that increases pH sensitivity of the polyaniline). The doped polyaniline sensor detects ammonia in the range of 25ppb-25ppm. The dopant is any type of protonic acid (e.g., camphor sulfonic acid).

0057 Some embodiments provide for an ammonia specific fuel cell sensor with anode and cathode electrodes and an anion exchange membrane (AEM) which is built to circulate hydroxide (OH) ions; wherein ammonia at the anode binds to hydroxide, oxygen (O₂) binds with H₂O at the cathode, wherein ammonia with O₂ generate N₂ and H₂O and wherein the ammonia fuel cell sensor generates voltage which increases in response to increased amount of ammonia and wherein the ammonia fuel cell sensor has sensitivity and specificity to ammonia gas with low limit of detection of ammonia at 2.6 ppb.

**0058** Some embodiments provide for an electrochemical sensor which is fabricated with methane selective material that has a resistivity that increases in response to increased concentration of methane (e.g., vanadium oxide (VO₂) mixed with nafion is spin coated on interdigitated electrodes). Wherein the methane specific material has a resistivity which increases in response to the increased amount of methane, wherein 200ppm of methane gas adsorbed on the VO₂/nafion films generate 15% of change in resistivity, and wherein the lowest limit of detection is 1ppm of methane.

**0059** Some embodiments provide for a breath test method used in connection with a handheld, portable breath analyzer. The method includes steps of: (a) providing a portable and hand-held breath analyzer, (b) prompting a subject to exhale a fasting breath sample into a removable mouthpiece which attaches removably to the receptacle on the main body of the breath analyzer, (c) allowing a microcontroller to measure resistivity of the sensor that occurs when the fasting breath sample contacts the sensor, (d) allowing the processor to measure a resistivity of the sensor that occurs when the fasting breath sample contacts the sensor, e) allowing the processor to convert the resistivity of the sensor to units (e.g., ppb) of gas and f) display the result on the screen.

**0060** Some embodiments provide a breath test method in connection with a handheld, portable breath analyzer which does not contain NaOH crystals or any type of desiccant or molecular sieve to remove humidity from the breath and allows the breath to contain 94%-97% of humidity.

**0061** Some embodiments provide a method of detecting ammonia in a person's fasting breath, the method comprising collecting a fasting breath sample from a subject, determining an amount of ammonia present in the fasting breath sample, and designating the presence of non-alcoholic steatohepatitis (NASH) if the amount of ammonia present in the fasting breath sample exceeds a predetermined value of breath ammonia. In some cases, collecting fasting breath sample from a subject comprises collecting the fasting breath sample into a single portable breath analyzer. In some cases, the determining an amount of ammonia present in the fasting breath sample comprises exposing the fasting breath sample to a sensor having a resistivity that increases in response to increased presence of ammonia and measuring resistivity of the fasting breath sample.

**0062** Some embodiments provide a method of detecting ammonia in a subject's breath, the method comprising collecting a breath sample from a subject, determining an amount of ammonia present in the breath sample, and designating presence of kidney disease in a subject, the method comprising collecting a breath sample from a subject, determining an amount of ammonia present in the breath sample, and designating a presence of kidney disease in the subject if the amount of ammonia present in the breath sample exceeds the amount of ammonia from a predetermined value. In some cases, the collecting a breath sample from a subject comprises collecting the breath sample from a single portable breath analyzer. In some cases, the determining an amount of ammonia present in the breath sample comprises exposing the breath sample to a sensor having a resistivity that increases in response to increased presence of ammonia and measuring resistivity of the breath sample. In some cases, the determining an amount of ammonia present in the breath sample comprises exposing the breath sample to a sensor generating voltage that increases in response to increased presence of ammonia, measuring voltage of the breath sample and converting the voltage to ammonia units (e.g., ppb, ppm).

**0063** Some embodiments provide a method of detecting methane in a subject's breath, the method comprising collecting a fasting breath sample from a subject, determining an amount of methane present in the fasting breath sample; ingesting a predetermined dose of carbohydrate (preferably lactulose) and collecting a breath samples at 30 minutes and at 60 minutes after the ingestion of lactulose and designating a presence of SIBO in the digestive tract if the amount of methane present in the post-ingestion breath samples exceeds the amount of methane present in the fasting breath sample by a predetermined value. In some cases, the collecting a baseline breath sample from a subject and the collecting a post-ingestion breath sample from the subject comprises collecting both the baseline breath sample and the post-ingestion breath sample from a single portable breath analyzer. In some cases, the determining an amount of methane present in the baseline breath sample comprises exposing the baseline breath sample to a sensor having a resistivity that increases in response to increased presence of methane and measuring resistivity of the baseline breath sample, and wherein the determining a concentration of methane present in the post-ingestion breath sample comprises exposing the post-ingestion breath sample to the sensor and measuring resistivity of the post-ingestion breath sample.

### DETAILED DESCRIPTION

**0064** The following detailed description is to be read with reference to the drawings. The drawings, which are not necessarily to scale, depict selected embodiments and are not intended to limit the scope of the invention. Skilled artisans will recognize that the examples provided herein have many useful alternatives that fall within the scope of the invention.

**0065** The present invention provides improved breath analyzer and breath test methods to determine whether a subject's breath contains higher than normally encountered levels of ammonia or methane determining whether the subject is affected by liver disease such as non-alcoholic fatty liver disease, or non-alcoholic steatohepatitis, or chronic liver disease, or cirrhosis, or chronic kidney disease, or metabolic disease, or intestinal malabsorption, or small bowel bacterial overgrowth, or H. Pylori infection. The improved breath analyzer and breath test is less costly and more convenient than existing methods of testing.

**0066** In some embodiments, the breath analyzer **Figure 1** is stand-alone, fully integrated, sensitive and specific to gases contained in human breath, easy-to-use by lay people; wherein the small size allows the breath analyzer to be hand-held; wherein the function as a fully integrated breath analyzer device allows the device to collect the breath, direct the breath to the sensor, to analyze the sensor signal, to store information and to deliver result accurately, in real time, at any point between 0.1 minutes and 2 minutes; wherein the conditions within the breath analyzer are controlled, such as flow of breath is 7L/min, temperature is 98.6 F and relative humidity is 94%-97%; wherein the dimensions are 4 ¼ x 2 ½ x 1¾ inches (**Figure 1**); and wherein the device is manufactured using 3-D printer (ANYCUBIC S model) using plastic acrylonitrile butadiene styrene (ABS) as illustrated in **Figure 11****.**

0067 In some embodiments, the breath analyzer **Figure 1** is stand-alone, fully integrated, sensitive and specific to gases contained in human breath, accurate and easy-to-use by lay people; wherein the small size allows it to be hand-held; wherein the function as a fully integrated device allows it to collect the breath, to analyze the breath, to store information and to deliver result accurately, in real time, in 0-2 minutes; wherein the environment within the device is controlled under specific conditions such as flow of breath (7L/min), temperature (98.6 F) and relative humidity at less than 94%; wherein the dimensions are smaller or larger than 4 ¼ x 2 ½ x 1¾ inches; and wherein the device is manufactured using non-3D printing types of manufacturing.

0068 In some embodiments the breath analyzer is an ammonia breathalyzer (AB-Device) as in **Figure 1** and comprises a removable mouthpiece, and a body of the device; wherein the body of the device comprises: a channel, a sensor, an amplifier, an analog to digital converter (ADC), a microcontroller, a microSD card, a liquid crystal display (LCD) and a power source; wherein the power source is a 9V battery or a lithium-polymer (LiPo) battery; wherein the 9V or a LiPo battery is a replaceable battery or a rechargeable battery; wherein the removable mouthpiece comprises a first portion and a second portion, wherein the first portion is sized and shaped to accommodate a user's lips and the second portion is sized and shaped to removably attach to the receptacle of the main body **Figure 1****,** and wherein the second portion contains antimicrobial filter to decontaminate the breath sample from bacteria; wherein the power source is a replaceable battery; wherein the sensor comprises an ammonia selective material and a conductive material, wherein the ammonia selective material contacts the conductive material, wherein the ammonia selective material has a resistivity that increases in response to increased concentration of ammonia; wherein the microcontroller detects resistivity and uses the resistivity to calculate a concentration of ammonia in the breath, wherein the concentration of ammonia is displayed on the screen **Figure 1****,** wherein the conductive material can include a plurality of electrodes, e.g., interdigitated finger electrodes.

0069 In some embodiments the breath analyzer device is an ammonia breathalyzer device (AB-Device) **Figure 1** and comprises a single-use, removable mouthpiece and a main body; wherein the main body comprises: a channel, a sensor, an amplifier, an analog to digital converter (ADC), a microcontroller, a microSD card, a liquid crystal display (LCD) and a power source; wherein the removable mouthpiece comprises a first portion and a second portion, wherein the first portion is sized and shaped to accommodate a user's lips and the second portion is sized and shaped to removably attach to receptacle of the main body, and wherein the second portion contains antimicrobial filter; wherein the power source is a removable battery; wherein the sensor comprises a fuel cell system sensitive to ammonia, wherein the fuel cell system comprises of an anode electrode and a cathode electrode and an anion exchange membrane (AEM) **Figure 12****;** wherein the AEM is made of Polyvinylidene, fluoride-co-hexafluoropropylene (PVDF-co-HFP), or Per-Fluorinated- Sulfonic-Acid (PFSA), or Nafion, or Fumapem; wherein the ammonia fuel cell system generates voltage that increases in response to increased concentration of ammonia; wherein the microcontroller detects voltage and uses the voltage to calculate a concentration of ammonia in the breath sample which is displayed on the screen **Figure 1****.**

**0070** In some embodiments the ammonia breath analyzer device **Figure 1** is used for in vitro diagnostic test for the detection of ammonia as a marker for non-alcoholic steatohepatitis (NASH); wherein evidence in publication by Thomsen, Karen Louise, Francesco De Chiara, Krista Rombouts, Hendrik Vilstrup, Fausto Andreola, Rajeshwar P. Mookerjee, and Rajiv Jalan, titled "Ammonia: A novel target for the treatment of non-alcoholic steatohepatitis." Medical Hypotheses 113 (2018): 91-97, elevated breath ammonia detected with the use of AB-Device can become marker of early onset NASH; wherein the ammonia breath analyzer is used to monitor the level of breath ammonia in subjects with diseases such as chronic liver disease, chronic kidney disease, H. Pylori infection, metabolic diseases and others.

**0071** In some embodiments the breath analyzer **Figure 1** is a methane breath analyzer device and comprises a single-use, removable mouthpiece and a body of the device; wherein the body comprises: a channel, a sensor, an amplifier, an analog to digital converter (ADC), a microcontroller, a microSD card, a liquid crystal display (LCD) and a power source **Figure 2****;** wherein the removable mouthpiece comprises a first portion and a second portion; wherein the first portion is sized and shaped to accommodate a user's lips and the second portion is sized and shaped to removably attach to receptacle of the main body, and wherein the second portion contains antimicrobial filter; wherein the power source is a replaceable battery; wherein the sensor is a methane specific sensor and comprises of a methane selective material and a conductive material; wherein the methane selective material is vanadium oxide **Figure 16****;** wherein vanadium oxide is added to nafion which is also methane selective material; wherein vanadium oxide **Figure 16** is an inorganic compound which, because of its high oxidation state, is both amphoteric oxide and an oxidizing agent; wherein nafion **Figure 21** is a synthetic polymer with ionic properties and has excellent thermal and mechanical stability; and wherein the conductive material includes a plurality of electrodes e.g., interdigitated fingers **Figure 5****.**

**0072** In some embodiments the methane breath analyzer is used for in vitro diagnostic breath test for the detection of methane in breath of subjects as evidence of small intestinal bacterial overgrowth (SIBO); wherein the methane breath analyzer is used for in vitro diagnostic breath test for the detection of methane in breath of subjects with diseases such as intestinal carbohydrate malabsorption, metabolic diseases and others.

0073 In some embodiments the general operational flow is illustrated in **Figure 2****;** wherein the operational flow is designed to facilitate the flow of breath sample and the function of the electronic circuit of the ammonia breath analyzer; and wherein the operational flow **Figure 2** is used in other types of breath analyzers, e.g., methane breath analyzer.

0074 In some embodiments the breath analyzer **Figure 1** operates with the operational flow **Figure 2****;** wherein the operational flow of the breath analyzer consists of the following steps:
0075 Step 1: the breath analyzer device is turned on and off using the on/off switch;
0076 Step 2: the screen prompts the user to breathe (exhale) into the mouthpiece for 4-5 seconds continuously;
0077 Step 3: the breath moves from the mouthpiece through the device's channel, to the sensor; the sensor's signal (analog) advances to the microcontroller through the amplifier and the analog-to-digital converter (ADC);
0078 Step 4: data are saved in the microSD card;
0079 Step 5: data are recalled to the microcontroller;
0080 Step 6, data are calibrated and the result is calculated;
0081 Step 7: the result is displayed on the screen.

0082 After the result is displayed, the screen prompts the user to turn off the breath analyzer device.

0083 In some embodiments the operational flow **Figure 2** is designed for a breath analyzer device which detects ammonia;

0084 In some embodiments the operational flow **Figure 2** is designed for a breath analyzer device which detects methane;

**0085** In some embodiments the operational flow **Figure 2** is designed for a breath analyzer device which detects hydrogen and methane;

**0086** In some embodiments, the operational flow **Figure 2** is designed for a breath analyzer device, wherein the sensor is a potentiometric sensor;

**0087** In some embodiments, the operational flow **Figure 2** is designed for a breath analyzer device, wherein the sensor is an amperometric sensor;

**0088** In some embodiments, the operational flow **Figure 2** is designed for a breath analyzer device, wherein the sensor is a fuel cell sensor;

**0089** In some embodiments, the operational flow **Figure 2** is designed for a breath analyzer device, wherein the sensor is any type of sensor, sensitive to gases found in the human breath.

**0090** In some embodiments, the operational flow **Figure 2** is designed for a breath analyzer device wherein the ammonia sensor is a sensor based on polyaniline (PANI) **Figure 4****,** wherein PANI is doped with acids such as HCL or Camphor sulfonic acid (CSA) **Figure 5****,** or other types of protonic acids, and wherein PANI is oxidized with oxidants such as ammonium persulfate (APS) or other types of oxidants.

**0091** In some embodiments the operational flow **(****Figure 2****)** is designed for breath analyzer device wherein the ammonia sensor is an ammonia sensitive fuel cell sensor.

**0092** In some embodiments the operational flow **Figure 2** is designed for a breath analyzer device wherein the methane sensor is based on Vanadium oxide or other type of oxide added to a synthetic polymer, e.g., Nafion.

**0093** In some embodiments the operational flow **Figure 2** is designed for a breath analyzer device wherein one or more than one sensor are exposed to breath gases such as hydrogen, ammonia, methane and others.

0094 In some embodiments changes in gas concentration induce proportional changes in sensor conductance. Changes in ammonia concentration induce proportional changes in the ammonia sensor conductance; wherein the changes in conductance are processed by the microcontroller **Figure 2****;** wherein the processed data comprise the data acquisition circuit **Figure 2** of the breath analyzer device; wherein the data acquisition circuit digitizes the ammonia sensor conductance readings; wherein the data acquisition circuit is battery powered; wherein the battery is housed in the main body **Figure 11****.** The battery-powered data acquisition circuit consists of:
0095 amplifier;
0096 analog- to-digital converter (ADC);
0097 microcontroller;
0098 liquid-crystal display (LCD); and
0099 power regulator circuit.

0100 In some embodiments the amplifier converts the conductance into voltage, while simultaneously conditioning the sensor, generating at its output the voltage value, proportional to the conductance of the sensor.

0101 In some embodiments the voltage value is digitized by the ADC and the digitized code is read by the microcontroller.

0102 In some embodiments the microcontroller also controls the LCD, which displays step-by- step instructions to the user and, at the conclusion of the breath test, results of the test.

0103 In some embodiments the power regulator in the microcontroller provides voltage levels necessary for the circuit.

0104 In some embodiments, when the subject turns on the breath analyzer **Figure 1****,** a baseline measurement (no breath) of the sensor's signal is taken by the microcontroller to determine the baseline conductance of the sensor at the time prior to the subject exhaling into the breath analyzer device through the first portion of the mouthpiece, which is removably attached to the receptacle of the breath analyzer device **Figure 1****;** in some cases, the baseline measurement of the conductance by the microcontroller without breath takes place between 0 minutes and 0.1 minutes **Figure 3****;** after the baseline measurement is taken a message is shown on the display, which instructs the subject to exhale into the first portion of the mouthpiece **Figure 1** for 4 to 5 seconds; the subject exhales in the first portion of the mouthpiece for 4 to 5 seconds continuously; the microcontroller begins to calculate the sensor conductance after the subject finishes exhaling into the mouthpiece and calculates the peak value of the sensor conductance **Figure 3** after the subject finishes exhaling into the mouthpiece; in some cases, the peak occurs at 0.2 minutes to 0.4 minutes after the subject's exhalation into the mouthpiece ends; The microcontroller **Figure 2** calculates the difference between the measured peak value and the measured baseline value **Figure 3****;** the difference between the measured peak value and measured baseline is proportional to the concentration of the gas to which the breath analyzer is specific and the difference between the measured peak value and the measured baseline is reported as the result; the result is displayed on the screen of the breath analyzer device **Figure 1****.**

**0105** **Figure 3** illustrates examples of analysis of subjects' breath samples using the ammonia breath analyzer device **Figure 1****;** wherein the breath analyzer data processing circuit detects resistivity of the sensor which is converted in the concentration of ammonia in the breath samples. There are three types of breath samples **Figure 3****:** 1) breath samples from subjects who fasted for 9 hours (graph labeled "fasting"); 2) breath samples from subjects 30 minutes after they ingested a post-9 hr.-fasting meal (graph labeled "30 min post-meal"); and 3) breath samples from subjects who are at half time between the end of ingesting one meal and the beginning of ingesting the next meal (graph labeled "between meals"). A fourth graph labeled "no breath" illustrates the breath analyzer's **Figure 1** sensor signal processing when no subject exhales into the mouthpiece and no breath reaches the sensor (dry run); wherein the straight line labeled "no breath" illustrates the stability of the ammonia sensor in the ammonia breath analyzer device.

**0106** In some embodiments the analog to digital converter (ADC) code is proportional to the resistance of the sensor, which is related to the ammonia concentration according to the following formula: y = 5E- 07x3 - 9E-05x2 + 0.0047x + 0.021 based on the ammonia sensor calibration curve.

**0107** In some embodiments the sensor in the breath analyzer device **Figure 1** is an ammonia specific sensor based on polyaniline (PANI). PANI **Figure 4** was first reported in 1862, and is one of the most investigated and widely used conducting polymers due to its facile synthesis in acidic aqueous solutions, environmental stability, inexpensive monomer, good processibility and solubility in common organic solvents, which allow it to be blended with other polymers; wherein PANI exhibits three different oxidation states: leucoemeraldine (LEB, fully reduced), emeraldine (EB, half-oxidized) and pernigraniline (PNB, fully oxidized); wherein the emeraldine salt, the protonated form of EB, is the only conducting form and is usually obtained by protonation of the basic amine and imine sites in EB with strong acids. This is an important characteristic of PANI, especially for ammonia detection, as it deprotonates the amine groups in the emeraldine salt converting it to the emeraldine base form with a corresponding drop in conductivity of several orders of magnitude **Figure 4****.** The reaction that allows this change in conductivity is as follows: *PANI*+ + *NH₃* 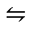 *PANI* + *NH₄+*

**0108** In some embodiments, the ammonia sensor **Figure 5** is fabricated as follows: PANI doped with HCl is prepared by chemical oxidative polymerization of aniline in aqueous acidic medium (1M HCl) with ammonium persulfate (APS) as an oxidant. The mixture is left to polymerize overnight at room temperature. The PANI precipitate is collected on a filter paper and washed repeatedly with 0.1M HCl followed by repeated washes with acetone. Deprotonation of the resulting PANI salt is performed by stirring the powder in an aqueous 0.1M NH₄OH solution for 24 hrs. at room temperature thus obtaining the emeraldine base (EB), which is then washed with water repeatedly until neutral pH and then dried under vacuum for 48 hours at 60°C. PANI is re-doped with Camphor sulfonic acid (CSA) with molar ratio of 2:1 to form PANI-CSA. Thin film interdigitated platinum electrodes (IDA) (line spacing 100µm) are purchased from the Electronic Design Center, Case Western University. The ammonia sensor films are prepared by spin coating the PANI-CSA solution on the IDA electrodes. Prior to spin coating, the electrodes are cleaned by rinsing with methanol followed by rinsing with deionized water and drying in a stream of dry nitrogen. PANI-CSA films are spun-coated onto the IDA **Figure 5****.** Other acids used to dope PANI are sulfuric acid, salicylic acid, acetic acid, citric acid, tartaric acid, oxalic acid, malonic acid, succinic acid, glutamic acid, adipic acid, and phthalic acid.

**0109** In some embodiments the sensitivity of the PANI-CSA ammonia sensor is assessed by calibration; wherein calibration is performed using various concentrations of ammonia gas and measuring the sensor's resistivity in units of electrical resistivity (current), ohms (Ω) **Figure 6****.** Calibration is performed by making serial, 10-fold dilutions of ammonia gas starting at 25ppm ammonia to 2.5 ppm ammonia, to 250 ppb ammonia and to 25 ppb in tedlar bags. A 20cc syringe is used to collect the gas sample from the tedlar bag and then inject the withdrawn gas sample directly over the sensor. The change in sensor resistivity, as measure of sensitivity to the infused ammonia gas, correlates to the change in current **Figure 6** generated by the sensor in response to sensing of various concentrations of ammonia gas.

**0110** In some embodiments the specificity of the PANI-CSA sensor is assessed by exposing the ammonia sensor to atmospheric air, to nitrogen (N₂) and to CO₂; wherein N₂ and CO₂ are components of the human breath. Exposure of the PANI-CSA ammonia sensor to N₂, CO₂ and air demonstrates the sensor's high specificity to ammonia when compared to CO₂, N₂ and atmospheric air **Figure 7****.** The potential interference on the PANI-CSA sensor by CO₂, contained in exhaled breath, at 4%-5% and by N₂ at 79%, contained in exhaled breath, is tested when the PANI-CSA sensor is exposed to 100% CO₂, to 100% N₂, to atmospheric air and to 25ppm of ammonia which correlates to 0.000025% of ammonia gas **Figure** 7. The PANI-CSA shows significantly higher specificity to 0.000025% NH₃ as compared to 100% CO₂ and 100% N₂ and to atmospheric air which contains 21% of oxygen (O₂); wherein the exhaled air contains 13% O₂; wherein the specificity is a measure of the change in resistivity of the PANI-CSA ammonia sensor **Figure 7****.**

**0111** The high specificity of the PANI-CSA sensor **Figure 5** to ammonia and the minor interference from CO₂, N₂ and air is illustrated in **Figure 7****.** The atmospheric air, which contains 21% of O₂ as opposed to the exhaled breath which contains 13% O₂, has practically no effect on the PANI-CSA sensor's resistivity. This demonstrates that the exhaled O₂ has no interference with ammonia detection by the PANI-CSA sensor which is placed in the breath analyzer device **Figure 1****.**

**0112** The humidity in exhaled human breath, naturally, ranges between 94% and 97%. Ammonia is a hydrophilic molecule and humidity can affect the response of the PANI-CSA to the concentration of ammonia in the breath sample to which the PANI-CSA sensor is exposed when the subject exhales into the mouthpiece **Figure 1****.** Application of molecular sieve desiccant (3A Sigma Aldrich) in the vicinity of the PANI-CSA sensor; wherein vicinity is inside the mouthpiece, or in-front-of the sensor, or on-top-of the sensor, or in another part of the body of the device **Figure 1****,** as ammonia is infused, decreases the resistivity/sensitivity of PANI-CSA to the concentration of ammonia by 50% **Figure 8****.** Application of another type of desiccant, sodium hydroxide (NaOH) crystals, with the infusion of 25ppm of ammonia gas and 100% CO₂ gas on the PANI-CSA ammonia sensor significantly decreases the resistivity of the PANI-CSA sensor **Figure 9****.** The significant decrease in resistivity of PANI-CSA sensor to ammonia and to CO₂ is due to the increased absorption of ammonia by the molecular sieve **Figure 8** and by the NaOH crystals **Figure 9****.**

**0113** In some embodiments, the ammonia specific breath analyzer device **Figure 1** with PANI-CSA sensor **Figure 5** does not contain NaOH desiccant crystals, or molecular sieve or any other type of desiccant anywhere inside the body of the device or inside the mouthpiece. In other cases, the breath analyzer device **Figure 1** with PANI-CSA sensor contains a limited amount of molecular sieve and a limited number of NaOH crystals.

**0114** In some embodiments, the breath analyzer **Figure 1** is an ammonia specific device and contains an ammonia specific fuel cell sensor **Figure 12****;** wherein the fuel cell ammonia specific breath analyzer device does not contain NAOH desiccant, or molecular sieve or any other type of desiccant anywhere inside the body of the device or inside the mouthpiece. In other cases, the fuel cell ammonia sensor breath analyzer **Figure 1** contains a limited amount of molecular sieve and a limited number of NaOH crystals.

**0115** In some embodiments, the breath analyzer device **Figure 1** contains PANI-CSA ammonia sensor; wherein the PANI-CSA ammonia sensor remains stable at 30 days, 60 days and 180 days; wherein the stability of the PANI-CSA ammonia sensor is defined as straight line signal response when tested with no breath as in graph labeled "no breath" **Figure 3****;** wherein the stability of the sensor depends on the stability of PANI, which remains stable for over 12 months, and on the life of the battery within the breath analyzer during the time the breath analyzer device is in use.

**0116** In some embodiments, the life of the battery is monitored by the breath analyzer device **Figure 1** microcontroller **Figure 2** and the status of the battery life is displayed on the screen of the breath analyzer device.

**0117** In some embodiments, the breath analyzer device **Figure 1** contains ammonia selective sensor, which is exposed to the breath sample during use of the breath analyzer by the subject. The sensor is required to return to baseline after the end of use of the breath analyzer by the subject. The sensor begins to return to its baseline (regenerate) when the device is turned off after each use. The regeneration of the sensor is defined as return of the sensor's signal to its baseline as in graph "no breath" **Figure 3** after use of the breath analyzer device by the subject. In some cases, the sensor reaches full regeneration (returns to baseline) approximately 15 minutes after the ammonia breath analyzer **Figure 1** is turned off.

**0118** The concentration of breath ammonia in healthy subjects ranges from 50ppb to 2000ppb (2ppm). The concentration of breath ammonia in non-healthy subjects is greater than 2000ppb (2ppm). Elevated concentrations of breath ammonia occur in diseases which cause disturbance of the excretion of urea which is produced in the human body through metabolism of ingested protein. Such diseases are liver diseases, kidney diseases, urea cycle defects, diseases of metabolism and others.

**0119** Some embodiments provide a breath test method. In some cases the breath test is an ammonia breath test (AB-Test) and includes the following steps: 1) after overnight fast (9 hrs.), the subject brushes his/her teeth 15 minutes prior to using the ammonia breath analyzer device **Figure 1****;** 2) the subject places a new, removable mouthpiece to the device's receptacle **Figure 1****;** 3) turns on the breath analyzer device using the on/off switch and follows the instructions on the screen **Figure 1****;** 4) when prompted to breathe into the mouthpiece, the subject exhales continuously into the mouthpiece for 4-5 seconds continuously; 5) the device takes measurements of the sensor conductance and calculates the difference between baseline and the peak values and converts the values in concentration of ammonia in the subject's breath; and 6) the result is displayed on the screen **Figure 1****;** 7)When the result is displayed, the subject is prompted to turn off the device. In some cases, the ammonia breath test's duration is 0-2 minutes.

**0120** In other cases, the breath test is an ammonia breath test and includes the following steps: 1) without fasting, the subject brushes his/her teeth 15 minutes prior to using the ammonia breath analyzer device **Figure 1****;** 2) the subject places a new, single-use mouthpiece to the device's receptacle **Figure 1****;** 3) turns on the breath analyzer device using the on/off switch and follows the instructions on the screen **Figure 1****;** 4) when prompted to breathe into the mouthpiece, the subject exhales continuously into the mouthpiece for 4-5 seconds; 5) the breath analyzer takes measurements of the sensor conductance and calculates the difference between baseline and the peak values and converts the values in concentration of ammonia in the subject's breath; and 6) the result is displayed on the screen **Figure 1****;** 7)When the result is displayed, the subject is prompted to turn off the device. In some cases, the ammonia breath test's duration is 2 minutes.

**0121** In some embodiments, the ammonia breath test determines the presence of non-alcoholic steatohepatitis (NASH) when the concentration of *fasting for 9 hrs.* breath ammonia in a subject undergoing the ammonia breath test using the ammonia breathalyzer device **Figure 1****,** is greater than a predetermined value.

**0122** In other embodiments, the ammonia breath test determines the presence of kidney disease if the *non-fasting* breath ammonia concentration in a subject undergoing the ammonia breath test using the ammonia breath analyzer device **Figure 1****,** is greater than a predetermined value.

0123 In other embodiments, the ammonia breath test determines the presence of metabolic diseases if the *fasting* and *non-fasting* breath ammonia concentration in a subject undergoing the ammonia breath test using the ammonia breath analyzer device **Figure 1****,** is greater than a predetermined value.

0124 In other embodiments, the ammonia breath test determines the presence of urea cycle defects if the *fasting* and *non-fasting* breath ammonia concentration in a subject undergoing the ammonia breath test using the breath analyzer device **Figure 1****,** is greater than a predetermined value.

0125 In some embodiments, the ammonia sensor is based on fuel cell system **Figure 12****,** which consists of an anode electrode, a cathode electrode and an anion exchange membrane (AEM) and provides highly specific sensing for ammonia. The anion exchange membrane (AEM) is sandwiched between the anode electrode and the cathode electrode with platinum (Pt/C) catalyst coated on both anode and cathode. Protons' transport across the AEM electrolyte membrane is theoretically constant with steady humidity and oxygen concentration in the cathode side, and the hydroxide transportation rate is determined during construction of the fuel cell. The AEM generates current flow between the anode and cathode electrodes when ammonia is present. In the ammonia fuel cell, **Figure 12****,** oxidation occurs with hydroxide in linear proportion to the concentration of ammonia, and generates proportional voltage which can be measured using a potentiometric analyzer such as CH Instrument 660D. The generated voltage increases in proportion to the concentration of ammonia in the fuel cell structure as illustrated in **Figure 15****.**

0126 Polyvinylidene fluoride-co-hexafluoropropylene (PVDF-co-HFP), Per-Fluorinated- Sulfonic-Acid (PFSA), Nafion, and Fumapem are materials used in the construction of AEM of the ammonia fuel cell sensor and are obtained from Fuel Cell Store (College Station, TX). Electrodes and catalysts are provided by Sigma Aldrich. Standard ammonia is prepared by mass flow base gas dilutor from EGas Depot (Clearwater, FL).

0127 The ammonia fuel cell sensor's performance is examined in a system consisting of the sensor, and a breath ammonia simulator, which contains a flow meter, moisture separator, data recorder, and computer analyzer. The breath ammonia simulator is constructed for the purpose of simulating human breath with respect to the breath's content of humidity and ammonia. When the breath ammonia simulated gas is transported through the ammonia simulator, a certain concentration of ammonia molecule is mixed with the standard air. The ammonia oxidation reaction occurs immediately as long as the ammonia mixture diffuses into the anode, generating protons and electrons and is demonstrated by the potentiometer. The concatenation and transportation properties of each compound involved in the fuel cell (gas, ion, liquid) are critical and are defined and measured in the system. The anode and cathode electrodes and the anion exchange membrane (AEM) determine the performance of the fuel cell sensor.

**0128** In other embodiments, the sensor characterization system (SCS) is constructed as illustrated in **Figure 10****.** The sensor is characterized under standard conditions such as air flow, temperature and humidity, to closely resemble the conditions of the human breath.

**0129** The chemical reactions within the fuel cell during exposure to ammonia are illustrated in **Figure 12****.** The baseline of the electrical potential difference, which is equivalent to voltage (dV), between anode and cathode electrodes is 0.75 mV **Figure 14****.** Exposure of the fuel cell **Figure 12** to 50ppm of ammonia generates 1.05 mV potential difference (dV) between anode and cathode electrodes with 50% signal change **Figure 14****;** exposure of the fuel cell **Figure 12** to 40ppm of ammonia generates 1mV potential difference (dV) between electrodes **Figure 14****;** exposure of the fuel cell **Figure 12** to 30ppm of ammonia generates 0.96mV potential difference (dV) between electrodes **Figure 14****;** and exposure of the fuel cell **Figure 12** to 15ppm of ammonia generates 0.9mV potential difference (dV) between electrodes **Figure 14****.**

**0130** Calibration of the ammonia fuel cell sensor **Figure 12** is performed using various concentrations of ammonia gas to measure the generated potential difference (dV). The ammonia fuel cell sensor **Figure 12** is exposed to 50ppm ammonia, 40ppm ammonia, 30ppm ammonia, 15ppm ammonia and 1ppm ammonia and the response of the fuel cell sensor in voltage is illustrated in **Figure 15****.** The regression of the calibration curve **Figure 15** yields a sensitivity of 0.01% dV% for 1ppm concentration of ammonia and ideal lower limit of detection (LOD) at 2.6 ppb of ammonia; wherein the dV% is a measure of sensitivity of the fuel cell sensor **Figure 12** to ammonia; and wherein the dV% increases in response to increasing concentration of ammonia.

**0131** In some embodiments, the breath analyzer device **Figure 1** is a methane breath analyzer and comprises a removable mouthpiece, and a body of the device; wherein the body of the device comprises: a channel, a sensor, an amplifier, an analog to digital converter (ADC), a microcontroller, a microSD card, a liquid crystal display (LCD) and a power source; wherein the power source is a 9V battery or a lithium-polymer (LiPo) battery; wherein the 9V or a LiPo battery is a replaceable battery or a rechargeable battery; wherein the removable mouthpiece comprises a first portion and a second portion, wherein the first portion is sized and shaped to accommodate a user's lips and the second portion is sized and shaped to removably attach to the receptacle of the main body **Figure 1****,** and wherein the second portion contains antimicrobial filter; wherein the sensor comprises a methane selective material and a conductive material; wherein the methane selective material is vanadium oxide (VO₂) added to nafion; wherein the methane selective (vanadium oxide/nafion) material contacts the conductive material; wherein the methane selective(vanadium oxide/nafion) material has a resistivity that increases in response to increased concentration of methane; wherein the microcontroller detects resistivity and uses the resistivity to calculate a concentration of methane in the breath, wherein the concentration of methane is displayed on the screen **Figure 1****;** wherein the conductive material can include a plurality of electrodes, e.g., interdigitated finger electrodes **Figure 5****.**

**0132** The VO₂/nafion methane sensor is constructed as follows: First, 0.3g vanadium oxide powder is dispersed ultrasonically in 15 mL ethyl alcohol or IPA for 30 min with an appropriate amount of dispersant, and mixed with 5 g Nafion (M.W. 90 000-120 000, Aladdin Chemistry Co., Ltd.). The VO₂/nafion mixture is uniformly cast onto wafer substrate with finger electrodes, which are platinum (Pt) interdigitated finger electrodes [IDE] pre-patterned on silica, by the spin-coating method with speed of 600 r/min for 20 s and then 1000 r/min for 20 s. After removing the liquid by drying in an oven at 80 °C for 60 min, the VO₂ nanoparticle-based thermochromic film is obtained and the VO₂/nafion sensor is fabricated. The VO₂/nafion sensor performance is examined in the electrochemical sensing system **Figure 13** consisting of a feed vessel which has stirring capabilities, peristaltic pump to advance nitrogen (N₂) into the stirring vessel, insulation from the environment, and a sensor array chamber. T1 and T2 are the temperatures in both ends of the system. Amperometry is applied to the interdigitated finger electrodes of the VO₂/nafion sensor.

**0133** The VO₂/nafion sensor is calibrated using the electrochemical sensing system **Figure 13****.** Precision gas diluter Model 1010 from Custom Sensor Solutions is used to transport methane gas into the array chamber which contains the VO₂/nafion sensor. Tedlar bags (5L, prest-O sales and 0.5L Zefon) are used to make the required dilution of methane gas with the precision gas diluter. The gas samples with a series of specific humidity are prepared into Tedlar bag and measured by the VO₂/nafion sensor immediately. Amprobe THWD- 5 analyzer is used to measure humidity externally (+/- 3 % from RH 10 % - 90 %). Teflon tubing is used to connect the gas to the flow setup to minimize any gas absorption.

**0134** The VO₂/nafion sensor generates current (amps) in response to exposure to methane. The generated current is proportional to the amount of methane adsorbed on the VO₂/nafion sensor. **Figure 19** illustrates the response of VO2/nafion sensor to various concentrations of methane. I-T amperometry is applied on the sensor with 0.5 V and N₂ is used as background. The sensor baseline (without exposure to methane) generates 2.55amps current; 100ppm methane generate 3.05 amps; 60ppm of methane generate 2.88amps; 50ppm of methane generate 2.86amps; 30ppm of methane generate 2.82 amps; 10ppm of methane generate 2.72amps; and 1ppm of methane generate 2.6amps. The lowest concentration of methane detected by the VO₂/nafion sensor is 1ppm.

**0135** The VO₂/nafion generated current is converted to resistivity. Resistivity is the measure for sensitivity of the VO2/nafion sensor to methane and is illustrated in **Figure 20****.** The VO₂/nafion sensor's resistivity increases with increasing concentration of methane. Various concentrations of methane are tested on the VO₂/nafion film which demonstrate that the change in dR/Ro is 15% after exposure to 200ppm methane gas and the minimum concentration of methane tested is 1ppm. The calibration curve of methane using VO₂/nafion sensor is plotted in **Figure 20****.** The regression of the methane calibration curve has R²=0.9933.

**0136** Some embodiments provide a breath test method. In some cases the breath test is a methane breath test which determines the presence of small intestinal bacterial overgrowth (SIBO) in a subject and includes the following steps: 1) the subject fasts overnight (9 hrs.), 2) the subject brushes his/her teeth 15 minutes prior to using the methane breath analyzer; 2) the subject places a new, single-use mouthpiece to the device's receptacle **Figure 1****;** 3) turns on the breath analyzer device using the on/off switch and follows the instructions on the screen **Figure 1****;** 4) when prompted to breathe into the mouthpiece, the subject exhales continuously into the mouthpiece for 4-5 seconds; 5) the breath analyzer takes measurements of the sensor conductance and calculates the difference between baseline and the peak values and converts the values in concentration of methane in the subject's breath; and 6) the result is displayed on the screen **Figure 1****;** 7)When the result is displayed, the subject is prompted to turn off the device; 7) the subject ingests a predetermined amount of carbohydrate, preferably lactulose; 8) 30 minutes after ingestion of lactulose the subject follows steps 3-6 of the breath test method; 9) 60 minutes after ingestion of lactulose the subject follows steps 3-6 of the breath test method. At the end of the 60-minute test the breath analyzer through its microcontroller calculates the difference between the concentration of breath methane at 30-minute and the baseline (fasting) concentration of breath methane, and the difference between the 60-minute and the baseline concentration of breath methane. The breath test is determined to be indicative of SIBO if the difference is greater than a predetermined value.

## Claims

1. A handheld, portable breath analyzer, comprising:
a main body, which includes a channel, a sensor, an electronic circuit and a power source; and
a removable, mouthpiece, which is single-use and removably attaches to the main body;
wherein the channel accepts the breath sample and directs the breath sample to the sensor;
wherein the sensor contacts the breath sample; wherein the breath sample, naturally, contains humidity at 94%-97%; and wherein the humidity is maintained within the breath analyzer; wherein no desiccant is used to block the natural humidity of the human breath;
wherein the sensor comprises an ammonia sensitive system; wherein the ammonia sensitive system is a fuel cell system comprising an anode electrode, a cathode electrode and an anion exchange membrane; wherein the anion exchange membrane (AEM) is sandwiched between the anode electrode and the cathode electrode;
wherein the fuel cell system has an electric potential difference that increases in response to increased concentrations of ammonia;
wherein the electronic circuit comprises an amplifier, an analog to digital converter (ADC), a microcontroller, a microSD card, a liquid crystal display (LCD), and a power regulator circuit;
wherein the amplifier converts the sensor's conductance into voltage, while simultaneously conditioning the sensor generating at its output the voltage value, proportional to the conductance of the sensor; wherein the voltage value is digitized by the ADC and the digitized code is read by the microcontroller; wherein the microcontroller also controls the LCD; wherein the power regulator provides voltage levels necessary for the circuit; and wherein the microcontroller converts voltage to concentration of ammonia and displays the result on the screen.

2. The breath analyzer of claim 1 wherein the anion exchange membrane is constructed with polyvinylidene fluoride-co-hexafluoropropylene (PVDF-co-HFP), or with Per-Fluorinated-Sulfonic-Acid (PFSA), or with Nafion or with Fumapem.

3. The breath analyzer of claim 1 wherein the baseline of electric potential difference (dV) between anode and cathode electrodes is 0.7 mV.

4. The breath analyzer of claim 1 wherein the electric potential difference (dV) between anode and cathode electrodes becomes1.05 mV after exposing the fuel cell system to 50 ppm ammonia; wherein the fuel cell signal withstands 50% change.

5. The breath analyzer of claim 1 wherein the lower limit of detection of ammonia is 2.6ppb of ammonia.

6. The breath analyzer of claim 1 wherein the removable mouthpiece comprises a first portion and a second portion, wherein the first portion is sized and shaped to accommodate a user's lips and the second portion is sized and shaped to removably attach to receptacle of the main body, and wherein the second portion contains antimicrobial filter.

7. A hand-held, portable breath analyzer, which comprises:
a main body which includes a channel, a sensor, an electronic circuit, and a power source; and
a removable, single-use, mouthpiece, which comprises a first portion and a second portion, wherein the first portion is sized and shaped to accommodate a user's lips and the second portion is sized and shaped to removably attach to receptacle of the main body, and wherein the second portion contains antimicrobial filter;
wherein the channel accepts the breath sample and directs the breath sample to the sensor;
wherein the electronic circuit comprises an amplifier, an analog to digital converter (ADC), a microcontroller, a microSD card, a liquid crystal display (LCD), and a power regulator circuit;
wherein the sensor comprises an ammonia selective material and a conductive material; wherein the ammonia selective material is doped poly aniline (PANI), and the conductive material is a plurality of platinum interdigitated fingers, each 100µm apart;
wherein the sensor accepts the breath sample, which naturally contains 94%-97% humidity; wherein the natural humidity (94%-97%) is not removed; and wherein no desiccant or other humidity removing material is present in the mouthpiece or the main body;
wherein polyaniline is doped with HCL and re-doped with camphor sulfonic acid (CSA) to fabricate PANI-CSA sensor; wherein the PANI-CSA sensor has a resistivity which increases with the increase of concentration of ammonia; wherein the resistivity is a measure of sensitivity of the sensor to ammonia; wherein the resistivity is converted by the microcontroller to concentration of ammonia and is reported as result.

8. The breath analyzer of claim 7 wherein the dopant comprises a protonic acid.

9. The breath analyzer of claim 8 wherein the dopant comprises a protonic acid selected from the group consisting of hydrochloric acid, sulfuric acid, salicylic acid, acetic acid, citric acid, tartaric acid, oxalic acid, malonic acid, succinic acid, glutamic acid, adipic acid, phthalic acid and camphor sulfonic acid.

10. A handheld, portable breath analyzer, comprising:
a main body, which includes a channel, a sensor, an electronic circuit and a power source; and
a single-use, removable mouthpiece, which comprises a first portion and a second portion, wherein the first portion is sized and shaped to accommodate a user's lips and the second portion is sized and shaped to removably attach to receptacle of the main body, and wherein the second portion contains antimicrobial filter;
wherein the channel accepts the breath sample and directs the breath sample to the sensor;
wherein the electronic circuit comprises an amplifier, an analog to digital converter (ADC), a microcontroller, a microSD card, a liquid crystal display (LCD), and a power regulator circuit;
wherein the sensor contacts the breath sample; wherein the breath sample, naturally, contains humidity at 94%-97%; wherein the natural humidity is not removed; wherein the sensor comprises methane selective material and conductive material; wherein the methane selective material comprises vanadium oxide (VO₂) mixed with nafion; wherein the conductive material is platinum (Pt) interdigitated electrodes (IDE) pre-patterned on silica; and wherein the methane selective material has a resistivity which increases in response to increased concentration of methane;
wherein the microcontroller converts resistivity to concentration of methane and displays the result on the screen.

11. The breath analyzer of claim 10 wherein the mixture of nanostructured metallic vanadium oxide and nafion (VO₂/nafion) is spin coated on wafer substrate.

12. The breath analyzer of claim 11 wherein the wafer substrate consists of platinum (Pt) interdigitated fingers.

13. The breath analyzer of claim 12 wherein I-T amperometry applied on the VO₂/nafion sensor measures 0.5 V with N₂ as background gas.

14. The breath analyzer of claim 13 wherein the minimum detection of methane is 1ppm.

15. A breath test method comprising the following steps:
A. providing a portable and handheld breath analyzer device which consists of:
a main body which includes a sensor, an electronic circuit and a power source; and
a single-use, removable mouthpiece which accepts and directs the breath sample to the sensor;
wherein the removable mouthpiece comprises a first portion and a second portion, wherein the first portion is sized and shaped to accommodate a user's lips and the second portion is sized and shaped to removably attach to receptacle of the main body, and wherein the second portion is coated with antimicrobial filter;
wherein the sensor contacts the breath sample, which naturally, contains humidity at 94%-97%; and wherein the sensor has a sensitivity to ammonia which increases with the increase of ammonia concentration; and wherein the sensor has a lower limit of detection of ammonia at 2.6ppb.
wherein the electronic circuit comprises an amplifier, an analog to digital converter (ADC), a microcontroller, a microSD card, a liquid crystal display (LCD), and a power regulator circuit;
wherein the amplifier converts the conductance into voltage, while simultaneously conditioning the sensor generating at its output the voltage value, proportional to the conductance of the sensor, wherein the voltage value is digitized by the ADC and the digitized code is read by the microcontroller;
wherein the microcontroller also controls the LCD, which displays step-by-step instructions to the user, and, at the conclusion of the test, results of the test, and wherein the power regulator provides voltage levels necessary for the circuit.
B. instructing a subject to follow dietary rules e.g., fasting overnight (9 hours);
C. instructing a subject to brush teeth 15 minutes prior to taking the breath test;
D. instructing a subject to place the single-use, removable mouthpiece into the receptacle on the body of the breath analyzer;
E. instructing a subject to turn on the breath analyzer through the on/off switch and follow instructions displayed on the screen;
F. allowing the breath analyzer to prompt a subject to wait;
G. allowing the breath analyzer to prompt a subject to breathe into the removable mouthpiece, continuously, for 4-5 seconds;
H. allowing the breath analyzer to accept the subject's breath, and to advance the subject's breath to the sensor;
I. allowing the signal of the sensor to be analyzed through the amplifier, the ADC, and the microcontroller;
J. allowing the data to be saved to the microSD card;
K. allowing the data to be recalled to the microcontroller;
L. allowing the microcontroller to calibrate the data and calculate the result;
M. allowing the result to be displayed on the screen;
N. allowing the breath analyzer to prompt a subject to turn off the breath analyzer.

16. The method of claim 15 wherein the breath analyzer does not contain desiccant to remove the humidity from the breath sample.

17. The method of claim 15 allowing the breath analyzer to complete the breath test in a predetermined time from when the subject ends breathing into the mouthpiece.

18. The method of claim 15 allowing the breath analyzer to take baseline and peak measurements and calculate the difference between the baseline and the peak measurement.

19. The method of claim 15 wherein the sensor is a fuel cell sensor comprised an anode electrode, a cathode electrode and an anion exchange membrane (AEM) constructed with of polyvinylidene fluoride-co-hexafluoropropylene (PVDF-co-HFP), or Per-Fluorinated- Sulfonic-Acid (PFSA), or Nafion, or Fumapem.

20. The method of claim 15 wherein the sensor is a PANI-CSA sensor and comprises ammonia selective material and conductive material; wherein the ammonia selective material is PANI-CSA; wherein the conductive material is plurality of platinum interdigitated fingers 100µm apart; wherein the PANI-CSA sensor has a resistivity which increases with increasing concentrations of ammonia; and wherein the PANI-CSA sensor has a lower level of detection of ammonia at 25ppb.
wherein the ammonia sensor accepts the breath sample which naturally contains 94%-97% humidity; wherein the naturally contained humidity (94%-97%) is not removed.

21. The method of claim 15 wherein the sensor comprises methane selective material on wafer substrate; wherein the methane selective material is VO₂/nafion; and wherein the wafer substrate is platinum interdigitated fingers on silica.
